# EUROPEAN PATENT SPECIFICATION

(11) **EP 1 359 938 B1**
(45) Date of publication and mention of the grant of the patent: **16.03.2011**
(21) Application number: 02760986.6
(22) Date of filing: 24.01.2002
(51) Int. Cl.: A61K 39/395

(54) **METHOD FOR TREATING PSORIASIS BY USING AN IL-17D ANTAGONIST**
VERFAHREN ZUR BEHANDLUNG VON PSORIASIS UNTER VERWENDUNG EINES IL-17D ANTAGONISTEN
METHODE DE TRAITEMENT DU PSORIASIS UTILISANT UN ANTAGONISTE DE L'IL-17D

(30) Priority: 25.01.2001 US 264219 P
(43) Date of publication of application: 12.11.2003
(60) Divisional application: 10184830.7
(73) Proprietor: ZymoGenetics, Inc., Seattle, WA 98102 (US)
(72) Inventor: MOORE, Emma, E., Seattle, WA 98199 (US); FOLEY, Kevin, P., Cambridge, MA 02142 (US); MADDEN, Karen, L., Bellevue, WA 98004 (US); YAO, Yue, Kenmore, WA 98028 (US); PRESNELL, Scott, R., Tacoma, WA 98407 (US)
(74) Representative: MacLean, Martin Robert
(86) International application number: PCT/US2002/002244
(87) International publication number: WO 2002/102411

(56) References cited:
- WO-A-00/15798
- WO-A-00/42187
- WO-A-01/68859
- WO-A-99/46380
- TEUNISSEN MARCEL B M ET AL: "Interleukin-17 and interferon-gamma synergize in the enhancement of proinflammatory cytokine production by human keratinocytes." JOURNAL OF INVESTIGATIVE DERMATOLOGY, vol. 111, no. 4, October 1998 (1998-10), pages 645-649, XP002241100 ISSN: 0022-202X
- HOMEY B ET AL: "Up-regulation of macrophage inflammatory protein-3 alpha/CCL20 and CC chemokine receptor 6 in psoriasis." JOURNAL OF IMMUNOLOGY (BALTIMORE, MD.: 1950) UNITED STATES 15 JUN 2000, vol. 164, no. 12, 15 June 2000 (2000-06-15), pages 6621-6632, XP002241101 ISSN: 0022-1767
- WONGPIYABOVORN JONGKONNEE ET AL: "Up-regulation of interleukin-13 receptor alpha1 on human keratinocytes in the skin of psoriasis and atopic dermatitis." JOURNAL OF DERMATOLOGICAL SCIENCE OCT 2003 LNKD- PUBMED:14527737, vol. 33, no. 1, October 2003 (2003-10), pages 31-40, ISSN: 0923-1811
- SPADARO A ET AL: "Interleukin 13 in synovial fluid and serum of patients with psoriatic arthritis." ANNALS OF THE RHEUMATIC DISEASES FEB 2002 LNKD- PUBMED:11796407, vol. 61, no. 2, February 2002 (2002-02), pages 174-176, ISSN: 0003-4967
- DEROCQ J M ET AL: "Interleukin-13 stimulates interleukin-6 production by human keratinocytes" FEBS LETTERS, ELSEVIER, AMSTERDAM, NL LNKD- DOI:10.1016/0014-5793(94)80601-2, vol. 343, no. 1, 18 April 1994 (1994-04-18), pages 32-36, XP025604642 ISSN: 0014-5793 [retrieved on 1994-04-18]

## Description

Psoriasis is one of the most common dermatologic diseases, affecting up to 1 to 2 percent of the world's population. It is a chronic inflammatory skin disorder characterized by erythematous, sharply demarcated papules and rounded plaques, covered by silvery micaceous scale. The skin lesions of psoriasis are variably pruritic. Traumatized areas often develop lesions of psoriasis. Additionally, other external factors may exacerbate psoriasis including infections, stress, and medications, *e.g*. lithium, beta blockers, and anti-malarials.

The most common variety of psoriasis is called plaque type. Patients with plaque-type psoriasis will have stable, slowly growing plaques, which remain basically unchanged for long periods of time. The most common areas for plaque psoriasis to occur are the elbows knees, gluteal cleft, and the scalp. Involvement tends to be symmetrical. Inverse psoriasis affects the intertriginous regions including the axilla, groin, submammary region, and navel, and it also tends to affect the scalp, palms, and soles. The individual lesions are sharply demarcated plaques but may be moist due to their location. Plaque-type psoriasis generally develops slowly and runs an indolent course. It rarely spontaneously remits.

Eruptive psoriasis (guttate psoriasis) is most common in children and young adults. It develops acutely in individuals without psoriasis or in those with chronic plaque psoriasis. Patients present with many small erythematous, scaling papules, frequently after upper respiratory tract infection with beta-hemolytic streptococci. Patients with psoriasis may also develop pustular lesions. These may be localized to the palms and soles or may be generalized and associated with fever, malaise, diarrhea, and arthralgias..

About half of all patients with psoriasis have fingernail involvement, appearing as punctate pitting, nail thickening or subungual hyperkeratosis. About 5 to 10 percent of patients with psoriasis have associated joint complaints, and these are most often found in patients with fingernail involvement. Although some have the coincident occurrence of classic Although some have the coincident occurrence of classic rheumatoid arthritis, many have joint disease (psoriatic arthritis) that falls into one of five type associated with psoriasis: (1) disease limited to a single or a few small joints (70 percent of cases); (2) a seronegative rheumatoid arthritis-like disease; (3) involvement of the distal interphalangeal joints; (4) severe destructive arthritis with the development of "arthritis mutilans"; and (5) disease limited to the spine.

A number of treatments exist for psoriasis but they do not result in satisfactory remission of the disease. Thus there is a need to discover new therapies that more effectively treat the disease.

The present invention meets this need by providing the use of an antagonist to a polypeptide selected from the group consisting of SEQ ID NOs: 2, 3, 4, 5, 7, 8, 10 and 11 for the manufacture of a medicament for the treatment of psoriasis wherein said antagonist is:
an antibody, antibody fragment or single-chain antibody that binds to said polypeptide;
a polypeptide selected from the group consisting of SEQ ID NOs: 13, 14, 15 and 16 or a subsequence thereof; or
an anti-sense nucleotide that binds to mRNA that encodes said polypeptide.

The present invention also provides an antagonist to a polypeptide selected from the group consisting of SEQ ID NOs: 2, 3, 4, 5, 7, 8, 10 and 11 for use in treating psoriasis wherein said antagonist is:
an antibody, antibody fragment or single-chain antibody that binds to said polypeptide;
a polypeptide selected from the group consisting of SEQ ID NOs: 13, 14, 15 and 16 or a subsequence thereof; or
an anti-sense nucleotide that binds to mRNA that encodes said polypeptide.

A preferred antagonist to IL-17D is the Membrane-Spanning Protein-5 (MSP-5), SEQ ID NOs.: 12 and 13, the mature extracellular portion of the polypeptide being comprised of SEQ ID NO: 14. A preferred embodiment is a soluble receptor SEQ ID NO:15, corresponding to amino acid residues 879-898 of SEQ ID NO:13, or the soluble receptor SEQ ID NO:16 corresponding to amino acid residues 856-875 of SEQ ID NO:13.

IL-17D is defined and methods for producing it and antibodies to IL-17D are contained in WO 00/15798, filed September 17,1999, and U.S. Patent Application No. 09/397,846 filed September 17, 1999. The polynucleotide and polypeptide of human IL-17D are represented by SEQ ID NOs: 1 - 8, and mouse IL-17D by SEQ ID NOs: 9-11. The MSP-5 sequences are SEQ ID NOs: 12-14, described in WO99/46380 and SEQ ID NOs: 15 and 16 are extracellular domains. Another inhibitor would be an anti-idiotypic antibody to MSP-5 that also binds to 1L-17D. The present application provides a method for down-regulating 1L-17D comprising administering an MSP-5 polypeptide that binds to IL-17D to an individual.

Molecular weights and lengths of polymers determined by imprecise analytical methods (*e.g*., gel electrophoresis) will be understood to be approximate values. When such a value is expressed as "about" X or "approximately" X, the stated value of X will be understood to be accurate to ±10%.

As used herein, the term "antibodies" includes polyclonal antibodies, affinity-purified polyclonal antibodies, monoclonal antibodies, and antigen-binding fragments, such as F(ab')₂ and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instances, humanized antibodies may retain non-human residues within the human variable region framework domains to enhance proper binding characteristics. Through humanizing antibodies, biological half-life may be increased, and the potential for adverse immune reactions upon administration to humans is reduced. The binding affinity of an antibody can be readily determined by one of ordinary skill in the art, for example, by Scatchard analysis. A variety of assays known to those skilled in the art can be utilized to detect antibodies that bind to protein or peptide. Exemplary assays are described in detail in Antibodies: A Laboratory Manual, Harlow and Lane (Eds.) (Cold Spring Harbor Laboratory Press, 1988). Representative examples of such assays include: concurrent immunoelectrophoresis, radioimmunoassay, radioimmunoprecipitation, enzyme-linked immunosorbent assay (ELISA), dot blot or Western blot assay, inhibition or competition assay, and sandwich assay.

### Use of Antagonist to IL-17D to Treat Psoriasis

As indicated in the discussion above and the examples below, IL-17D is involved in the pathology of psoriasis. The present invention relates in particular to use as defined in the claims for treating psoriasis using antagonists to IL-17D. The antagonists to IL-17D can be a soluble receptor such as SEQ ID NOs:15 and 16 that binds to IL-17D,or antibodies, single chain antibodies or fragments of antibodies that bind to IL-17D.

### Administration of Antagonists to IL-17D

The quantities of antagonists to IL-17D necessary for effective therapy will depend upon many different factors, including means of administration, target site, physiological state of the patient, and other medications administered. Thus, treatment dosages should be titrated to optimize safety and efficacy. Typically, dosages used *in vitro* may provide useful guidance in the amounts useful for *in vivo* administration of these reagents. Animal testing of effective doses for treatment of particular disorders will provide further predictive indication of human dosage. Methods for administration include oral, intravenous, peritoneal, intramuscular, transdermal or administration into the lung or trachea in spray form by means or a nebulizer or atomizer. Pharmaceutically acceptable carriers will include water, saline, buffers to name just a few. Dosage ranges would ordinarily be expected from 1µg to 1000µg per kilogram of body weight per day. A dosage of MSP-5 or an antibody that binds to IL-17D would be about 25 mg given twice weekly. For subcutaneous or intravenous administration of the antagonist to IL-17D, the antibody or MSP-5 can be in phosphate buffered saline. Also in skin diseases such as psoriasis, the antagonist to IL-17D can be administered via an ointment or transdermal patch. The doses by may be higher or lower as can be determined by a medical doctor with ordinary skill in the art. For a complete discussion of drug formulations and dosage ranges *see* Remington's Pharmaceutical Sciences, 18th Ed., (Mack Publishing Co., Easton, Penn., 1996), and Goodman and Gilman's: The Pharmacological Bases of Therapeutics, 9th Ed. (Pergamon Press 1996).

The invention is further illustrated by the following non-limiting examples.

### Example 1

### In situ Hybridization of IL-17D

Spatial distribution of *IL-17D* mRNA in normal and diseased skin tissues were studied using *in situ* hybridization analysis. In the skin sample analyzed, only psoriasis samples have keratinocyte signal. The hybridization results indicated that IL-17D was highly expressed in the skin of psoriasis patients, and to a lesser degree in the skin of patients with lichen planus.

### Example 2

### MSP-5 Binds to IL-17D

Two assays were used to determine that MSP-5 binds to IL-17D. The first was a secretion trap technique, which revealed that MSP-5 expressed from the human keratinocyte cell line, HaCAT bound to IL-17Dβ-9. This was confirmed by transfecting BHK cells with the *MSP-5* cDNA and showing that these transfected cells bound to iodinated IL-17Dβ-9

## Claims

1. Use of an antagonist to a polypeptide selected from the group consisting of SEQ ID NOs: 2, 3, 4, 5, 7, 8, 10 and 11 for the production of a medicament for the treatment of psoriasis, wherein said antagonist is an antibody, antibody fragment or single-chain antibody that binds to said polypeptide.

2. Use of an antagonist to a polypeptide selected from the group consisting of SEQ ID NOs: 2, 3, 4, 5, 7, 8, 10 and 11 for the production of a medicament for the treatment of psoriasis, wherein said antagonist is a polypeptide selected from the group consisting of SEQ ID NOs: 13, 14, 15 and 16 or a subsequence thereof.

3. Use of an antagonist to a polypeptide selected from the group consisting of SEQ ID NOs: 2, 3, 4, 5, 7, 8, 10 and 11 for the production of a medicament for the treatment of psoriasis, wherein said antagonist is an anti-sense nucleotide that binds to mRNA that encodes said polypeptide.

4. An antagonist to a polypeptide selected from the group consisting of SEQ ID NOs: 2, 3, 4, 5,7,8,10 and 11 for use in treating psoriasis, wherein said antagonist is an antibody, antibody fragment or single-chain antibody that binds to said polypeptide.

5. An antagonist to a polypeptide selected from the group consisting of SEQ ID NOs: 2, 3, 4, 5, 7, 8, 10 and 11 for use in treating psoriasis, wherein said antagonist is a polypeptide selected from the group consisting of SEQ ID NOs: 13, 14, 15 and 16 or a subsequence thereof.

6. Antagonist to a polypeptide selected from the group consisting of SEQ ID NOs: 2, 3, 4, 5, 7, 8, 10 and 11 for use in treating psoriasis, wherein said antagonist is an anti-sense nucleotide that binds to mRNA that encodes said polypeptide.

## Patentansprüche

1. Verwendung von einem Antagonisten für ein Polypeptid, ausgewählt aus der Gruppe, bestehend aus SEQ ID NRn.: 2, 3, 4, 5, 7, 8, 10 und 11, für die Herstellung von einem Medikament für die Behandlung von Psoriasis, wobei der Antagonist ein Antikörper, Antikörper-Fragment oder Single-Chain-Antikörper ist, der / das an das Polypeptid bindet.

2. Verwendung von einem Antagonisten für ein Polypeptid, ausgewählt aus der Gruppe, bestehend aus SEQ ID NRn.: 2, 3, 4, 5, 7, 8, 10 und 11, für die Herstellung von einem Medikament für die Behandlung von Psoriasis, wobei der Antagonist ein Polypeptid ist, ausgewählt aus der Gruppe, bestehend aus SEQ ID NRn.: 13, 14, 15 und 16 oder einer Sub-Sequenz davon.

3. Verwendung von einem Antagonisten für ein Polypeptid, ausgewählt aus der Gruppe, bestehend aus SEQ ID NRn.: 2, 3, 4, 5, 7, 8, 10 und 11, für die Herstellung von einem Medikament für die Behandlung von Psoriasis, wobei der Antagonist ein Anti-Sense-Nukleotid ist, das an mRNA bindet, die das Polypeptid codiert.

4. Antagonist für ein Polypeptid, ausgewählt aus der Gruppe, bestehend aus SEQ ID NRn.: 2, 3, 4, 5, 7, 8, 10 und 11, zur Verwendung beim Behandeln von Psoriasis, wobei der Antagonist ein Antikörper, Antikörper-Fragment oder Single-Chain-Antikörper ist, der / das an das Polypeptid bindet.

5. Antagonist für ein Polypeptid, ausgewählt aus der Gruppe, bestehend aus SEQ ID NRn.: 2, 3, 4, 5, 7, 8, 10 und 11, zur Verwendung beim Behandeln von Psoriasis, wobei der Antagonist ein Polypeptid ist, ausgewählt aus der Gruppe, bestehend aus SEQ ID NRn.: 13, 14, 15 und 16 oder einer Sub-Sequenz davon.

6. Antagonist für ein Polypeptid, ausgewählt aus der Gruppe, bestehend aus SEQ ID NRn.: 2, 3, 4, 5, 7, 8, 10 und 11, zur Verwendung beim Behandeln von Psoriasis, wobei der Antagonist ein Anti-Sense-Nukleotid ist, das an mRNA bindet, die das Polypeptid codiert.

## Revendications

1. Utilisation d'un antagoniste dirigé contre un polypeptide choisi dans le groupe comprenant SEQ ID NO : 2, 3, 4, 5, 7, 8, 10 et 11 pour la production d'un médicament destiné au traitement du psoriasis, où ledit antagoniste est un anticorps, un fragment d'anticorps ou un anticorps à chaîne simple qui se lie au dit polypeptide.

2. Utilisation d'un antagoniste dirigé contre un polypeptide choisi dans le groupe comprenant SEQ ID NO : 2, 3, 4, 5, 7, 8, 10 et 11 pour la production d'un médicament destiné au traitement du psoriasis, où ledit antagoniste est un polypeptide choisi dans le groupe comprenant SEQ ID NO : 13, 14, 15 et 16 ou une sous-séquence de celles-ci.

3. Utilisation d'un antagoniste dirigé contre un polypeptide choisi dans le groupe comprenant SEQ ID NO : 2, 3, 4, 5, 7, 8, 10 et 11 pour la production d'un médicament destiné au traitement du psoriasis, où ledit antagoniste est un nucléotide antisens qui se lie à l'ARNm qui code pour ledit polypeptide.

4. Antagoniste dirigé contre un polypeptide choisi dans le groupe comprenant SEQ ID NO : 2, 3, 4, 5, 7, 8, 10 et 11 pour une utilisation dans le traitement du psoriasis, où ledit antagoniste est un anticorps, un fragment d'anticorps ou un anticorps à chaîne simple qui se lie au dit polypeptide.

5. Antagoniste dirigé contre un polypeptide choisi dans le groupe comprenant SEQ ID NO : 2, 3, 4, 5, 7, 8, 10 et 11 pour une utilisation dans le traitement du psoriasis, où ledit antagoniste est un polypeptide choisi dans le groupe comprenant SEQ ID NO : 13, 14, 15 et 16 ou une sous-séquence de celles-ci.

6. Antagoniste dirigé contre un polypeptide choisi dans le groupe comprenant SEQ ID NO : 2, 3, 4, 5, 7, 8, 10 et 11 pour une utilisation dans le traitement du psoriasis, où ledit antagoniste est un nucléotide antisens qui se lie à l'ARNm qui code pour ledit polypeptide.


### SEQUENCE LISTING

<110> Foley, Kevin
   Moore, Emma E.
   Madden, Karen L.
   Yao, Yue
   Presnell, Scott R.
<120> Method for treating psoriasis
<130> 01-03PC
<150> 60/264,219
   <151> 2001-01-25
<160> 16
<170> FastSEQ for Windows Version 3.0
<210> 1
   <211> 1819
   <212> DNA
   <213> Homo sapiens
<220>
   <221> CDS
   <222> (71) ... (676)
<400> 1
   cgggcgcggg gcgcaggcgg gctcctccgg cgcgtgcgga cgctgagcgt ggcctgtccc      60
   tcaggtctgg atg ctg gta gcc ggc ttc ctg ctg gcg ctg ccg ccg agc        109
              Met Leu Val Ala Gly Phe Leu Leu Ala Leu Pro Pro Ser
               1               5                  10
   tgg gcc gcg ggc gcc ccg agg gcg ggc agg cgc ccc gcg cgg ccg cgg       157
   Trp Ala Ala Gly Ala Pro Arg Ala Gly Arg Arg Pro Ala Arg Pro Arg
       15                  20                  25
   ggc tgc gcg gac cgg ccg gag gag cta ctg gag cag ctg tac ggg cgc       205
   Gly Cys Ala Asp Arg Pro Glu Glu Leu Leu Glu Gln Leu Tyr Gly Arg
    30                  35                  40                  45
   ctg gcg gcc ggc gtg ctc agt gcc ttc cac cac acg ctg cag ctg ggg      253
   Leu Ala Ala Gly Val Leu Ser Ala Phe His His Thr Leu Gln Leu Gly
                       50                  55                  60
   ccg cgt gag cag gcg cgc aac gcg agc tgc ccg gca ggg ggc agg ccc      301
   Pro Arg Glu Gln Ala Arg Asn Ala Ser Cys Pro Ala Gly Gly Arg Pro
                   65                  70                  75
   gcc gac cgc cgc ttc cgg ccg ccc acc aac ctg cgc agc gtg tcg ccc      349
   Ala Asp Arg Arg Phe Arg Pro Pro Thr Asn Leu Arg Ser Val Ser Pro
               80                  85                  90
   tgg gcc tac aga atc tcc tac gac ccg gcg agg tac ccc agg tac ctg      397
   Trp Ala Tyr Arg Ile Ser Tyr Asp Pro Ala Arg Tyr Pro Arg Tyr Leu
           95                  100                 105
   cct gaa gcc tac tgc ctg tgc cgg ggc tgc ctg acc ggg ctg ttc ggc      445
   Pro Glu Ala Tyr Cys Leu Cys Arg Gly Cys Leu Thr Gly Leu Phe Gly
   110                 115                 120                 125
   gag gag gac gtg cgc ttc cgc agc gcc cct gtc tac atg ccc acc gtc      493
   Glu Glu Asp Val Arg Phe Arg Ser Ala Pro Val Tyr Met Pro Thr Val
                   130                 135                 140
   gtc ctg cgc cgc acc ccc gcc tgc gcc ggc ggc cgt tcc gtc tac acc      541
   Val Leu Arg Arg Thr Pro Ala Cys Ala Gly Gly Arg Ser Val Tyr Thr
               145                 150                 155
   gag gcc tac gtc acc atc ccc gtg ggc tgc acc tgc gtc ccc gag ccg      589
   Glu Ala Tyr Val Thr Ile Pro Val Gly Cys Thr Cys Val Pro Glu Pro
           160                 165                 170
   gag aag gac gca gac agc atc aac tcc agc atc gac aaa cag ggc gcc      637
   Glu Lys Asp Ala Asp Ser Ile Asn Ser Ser Ile Asp Lys Gln Gly Ala
       175                 180                 185
   aag ctc ctg ctg ggc ccc aac gac gcg ccc gct ggc ccc tgaggccggt       686
   Lys Leu Leu Leu Gly Pro Asn Asp Ala Pro Ala Gly Pro
   190                 195                 200
   cctgccccgg gaggtctccc cggcccgcat cccgaggcgc ccaagctgga gccgcctgga    746
   gggctcggtc ggcgacctct gaagagagtg caccgagcaa accaagtgcc ggagcaccag    806
   cgccgccttt ccatggagac tcgtaagcag cttcatctga cacgggaatc cctggcttgc     866
   ttttagctac aagcaagcag cgtggctgga agctgatggg aaacgacccg gcacgggcat     926
   cctgtgtgcg gcccgcatgg agggtttgga aaagttcacg gaggctccct gaggagcctc     986
   tcagatcggc tgctgcgggt gcagggcgtg actcaccgct gggtgcttgc caaagagata    1046
   gggacgcata tgctttttaa agcaatctaa aaataataat aagtatagcg actatatacc    1106
   tacttttaaa atcaactgtt ttgaatagag gcagagctat tttatattat caaatgagag    1166
   ctactctgtt acatttctta acatataaac atcgtttttt acttcttctg gtagaatttt    1226
   ttaaagcata attggaatcc ttggataaat tttgtagctg gtacactctg gcctgggtct    1286
   ctgaattcag cctgtcaccg atggctgact gatgaaatgg acacgtctca tctgacccac    1346
   tcttccttcc actgaaggtc ttcacgggcc tccaggtgga ccaaagggat gcacaggcgg    1406
   ctcgcatgcc ccagggccag ctaagagttc caaagatctc agatttggtt ttagtcatga    1466
   atacataaac agtctcaaac tcgcacaatt ttttccccct tttgaaagcc actggggcca     1526
   atttgtggtt aagaggtggt gagataagaa gtggaacgtg acatctttgc cagttgtcag    1586
   aagaatccaa gcaggtattg gcttagttgt aagggcttta ggatcaggcc gaatatgagg    1646
   acaaagtggg ccacgttagc atctgcagag atcaatctgg aggcttctgt ttctgcattc    1706
   tgccacgaga gctaggtcct tgatcttttc tttagattga aagtctgtct ctgaacacaa    1766
   ttatttgtaa aagttagaag ttctttttta aatcattaaa agaggcttgc tga          1819
<210> 2
   <211> 202
   <212> PRT
   <213> Homo sapiens
<400> 2
   Met Leu Val Ala Gly Phe Leu Leu Ala Leu Pro Pro Ser Trp Ala Ala
   1               5                   10                  15
   Gly Ala Pro Arg Ala Gly Arg Arg Pro Ala Arg Pro Arg Gly Cys Ala
               20                  25                  30
   Asp Arg Pro Glu Glu Leu Leu Glu Gln Leu Tyr Gly Arg Leu Ala Ala
           35                  40                  45
   Gly Val Leu Ser Ala Phe His His Thr Leu Gln Leu Gly Pro Arg Glu
       50                  55                  60
   Gln Ala Arg Asn Ala Ser Cys Pro Ala Gly Gly Arg Pro Ala Asp Arg
   65                  70                  75                  80
   Arg Phe Arg Pro Pro Thr Asn Leu Arg Ser Val Ser Pro Trp Ala Tyr
                   85                  90                  95
   Arg Ile Ser Tyr Asp Pro Ala Arg Tyr Pro Arg Tyr Leu Pro Glu Ala
               100                 105                 110
   Tyr Cys Leu Cys Arg Gly Cys Leu Thr Gly Leu Phe Gly Glu Glu Asp
           115                 120                 125
   Val Arg Phe Arg Ser Ala Pro Val Tyr Met Pro Thr Val Val Leu Arg
       130                 135                 140
   Arg Thr Pro Ala Cys Ala Gly Gly Arg Ser Val Tyr Thr Glu Ala Tyr
   145 150 155 160
   Val Thr Ile Pro Val Gly Cys Thr Cys Val Pro Glu Pro Glu Lys Asp
   165 170 175
   Ala Asp Ser Ile Asn Ser Ser Ile Asp Lys Gln Gly Ala Lys Leu Leu
   180 185 190
   Leu Gly Pro Asn Asp Ala Pro Ala Gly Pro
   195 200
<210> 3
   <211> 187
   <212> PRT
   <213> Homo sapiens
<400> 3
   Ala Gly Ala Pro Arg Ala Gly Arg Arg Pro Ala Arg Pro Arg Gly Cys
   1 5 10 15
   Ala Asp Arg Pro Glu Glu Leu Leu Glu Gln Leu Tyr Gly Arg Leu Ala
   20 25 30
   Ala Gly Val Leu Ser Ala Phe His His Thr Leu Gln Leu Gly Pro Arg
   35 40 45
   Glu Gln Ala Arg Asn Ala Ser Cys Pro Ala Gly Gly Arg Pro Ala Asp
   50 55 60
   Arg Arg Phe Arg Pro Pro Thr Asn Leu Arg Ser Val Ser Pro Trp Ala
   65 70 75 80
   Tyr Arg Ile Ser Tyr Asp Pro Ala Arg Tyr Pro Arg Tyr Leu Pro Glu
   85 90 95
   Ala Tyr Cys Leu Cys Arg Gly Cys Leu Thr Gly Leu Phe Gly Glu Glu
   100 105 110
   Asp Val Arg Phe Arg Ser Ala Pro Val Tyr Met Pro Thr Val Val Leu
   115 120 125
   Arg Arg Thr Pro Ala Cys Ala Gly Gly Arg Ser Val Tyr Thr Glu Ala
   130 135 140
   Tyr Val Thr Ile Pro Val Gly Cys Thr Cys Val Pro Glu Pro Glu Lys
   145 150 155 160
   Asp Ala Asp Ser Ile Asn Ser Ser Ile Asp Lys Gln Gly Ala Lys Leu
   165 170 175
   Leu Leu Gly Pro Asn Asp Ala Pro Ala Gly Pro
   180 185
<210> 4
   <211> 186
   <212> PRT
   <213> Homo sapiens
<400> 4
   Gly Ala Pro Arg Ala Gly Arg Arg Pro Ala Arg Pro Arg Gly Cys Ala
   1 5 10 15
   Asp Arg Pro Glu Glu Leu Leu Glu Gln Leu Tyr Gly Arg Leu Ala Ala
   20 25 30
   Gly Val Leu Ser Ala Phe His His Thr Leu Gln Leu Gly Pro Arg Glu
   35 40 45
   Gln Ala Arg Asn Ala Ser Cys Pro Ala Gly Gly Arg Pro Ala Asp Arg
   50 55 60
   Arg Phe Arg Pro Pro Thr Asn Leu Arg Ser Val Ser Pro Trp Ala Tyr
   65 70 75 80
   Arg Ile Ser Tyr Asp Pro Ala Arg Tyr Pro Arg Tyr Leu Pro Glu Ala
   85 90 95
   Tyr Cys Leu Cys Arg Gly Cys Leu Thr Gly Leu Phe Gly Glu Glu Asp
   100 105 110
   Val Arg Phe Arg Ser Ala Pro Val Tyr Met Pro Thr Val Val Leu Arg
   115 120 125
   Arg Thr Pro Ala Cys Ala Gly Gly Arg Ser Val Tyr Thr Glu Ala Tyr
   130 135 140
   Val Thr Ile Pro Val Gly Cys Thr Cys Val Pro Glu Pro Glu Lys Asp
   145 150 155 160
   Ala Asp Ser Ile Asn Ser Ser Ile Asp Lys Gln Gly Ala Lys Leu Leu
   165 170 175
   Leu Gly Pro Asn Asp Ala Pro Ala Gly Pro
   180 185
<210> 5
   <211> 185
   <212> PRT
   <213> Homo sapiens
<400> 5
   Ala Pro Arg Ala Gly Arg Arg Pro Ala Arg Pro Arg Gly Cys Ala Asp
   1 5 10 15
   Arg Pro Glu Glu Leu Leu Glu Gln Leu Tyr Gly Arg Leu Ala Ala Gly
   20 25 30
   Val Leu Ser Ala Phe His His Thr Leu Gln Leu Gly Pro Arg Glu Gln
   35 40 45
   Ala Arg Asn Ala Ser Cys Pro Ala Gly Gly Arg Pro Ala Asp Arg Arg
   50 55 60
   Phe Arg Pro Pro Thr Asn Leu Arg Ser Val Ser Pro Trp Ala Tyr Arg
   65 70 75 80
   Ile Ser Tyr Asp Pro Ala Arg Tyr Pro Arg Tyr Leu Pro Glu Ala Tyr
                   85                  90                  95
   Cys Leu Cys Arg Gly Cys Leu Thr Gly Leu Phe Gly Glu Glu Asp Val
               100                 105                 110
   Arg Phe Arg Ser Ala Pro Val Tyr Met Pro Thr Val Val Leu Arg Arg
           115                 120                 125
   Thr Pro Ala Cys Ala Gly Gly Arg Ser Val Tyr Thr Glu Ala Tyr Val
       130                 135                 140
   Thr Ile Pro Val Gly Cys Thr Cys Val Pro Glu Pro Glu Lys Asp Ala
   145                 150                 155                 160
   Asp Ser Ile Asn Ser Ser Ile Asp Lys Gln Gly Ala Lys Leu Leu Leu
                   165                 170                 175
   Gly Pro Asn Asp Ala Pro Ala Gly Pro
               180                 185
<210> 6
   <211> 2361
   <212> DNA
   <213> Homo sapiens
<220>
   <221> CDS
   <222> (572)...(1202)
<400> 6
   gaattcggca cgagggtcag ggaagtattc agtgctttgt tgtagagttg ttggatagag      60
   gcacaggatc atttcatgtt gttgaggaga aaggagcaac agcctcctcc caccttatta     120
   aaaatagaga tttaaaaaaa cctctaattt cctcgaagta cagaatctca agaggtagct     180
   ctaaggagaa tccctctggg tttgagcgca ttcctcttcc agggggccta ttcttggact     240
   gctttcctta atagagaaat ctctctgagc caaaatcggc ctcccccaat tccatcctgt     300
   cggccccact tttctgctcc ggagacttcc aagccagtcc ccactcctcc ttcagccagt     360
   cgggcccgca cccgcgcccg gcagggccag ccctctcctc ctcctgcgtg gcgcagcaca     420
   ggccctgagc gcgcgacccc aggccctggg cgccccgccg catgctcgcg gctggaagcc     480
   ccagtttgcg tggcccttcg ggttattccg ctcaagagcc gccgcgtcgc cccatctcgg     540
   cgcgaatctg aaagcgcttt cgggggagaa g atg ttg ggg gca ctg gtc tgg        592
                                      Met Leu Gly Ala Leu Val Trp
                                      1               5
   atg ctg gta gcc ggc ttc ctg ctg gcg ctg ccg ccg agc tgg gcc gcg       640
   Met Leu Val Ala Gly Phe Leu Leu Ala Leu Pro Pro Ser Trp Ala Ala
           10                  15                  20
   ggc gcc ccg agg gcg ggc agg cgc ccc gcg cgg ccg cgg ggc tgc gcg       688
   Gly Ala Pro Arg Ala Gly Arg Arg Pro Ala Arg Pro Arg Gly Cys Ala
   25 30 35
   gac cgg ccg gag gag cta ctg gag cag ctg tac ggg cgc ctg gcg gcc 736
   Asp Arg Pro Glu Glu Leu Leu Glu Gln Leu Tyr Gly Arg Leu Ala Ala
   40 45 50 55
   ggc gtg ctc agt gcc ttc cac cac acg ctg cag ctg ggg ccg cgt gag 784
   Gly Val Leu Ser Ala Phe His His Thr Leu Gln Leu Gly Pro Arg Glu
   60 65 70
   cag gcg cgc aac gcg agc tgc ccg gca ggg ggc agg ccc gcc gac cgc 832
   Gln Ala Arg Asn Ala Ser Cys Pro Ala Gly Gly Arg Pro Ala Asp Arg
   75 80 85
   cgc ttc cgg ccg ccc acc aac ctg cgc agc gtg tcg ccc tgg gcc tac 880
   Arg Phe Arg Pro Pro Thr Asn Leu Arg Ser Val Ser Pro Trp Ala Tyr
   90 95 100
   aga atc tcc tac gac ccg gcg agg tac ccc agg tac ctg cct gaa gcc 928
   Arg Ile Ser Tyr Asp Pro Ala Arg Tyr Pro Arg Tyr Leu Pro Glu Ala
   105 110 115
   tac tgc ctg tgc cgg ggc tgc ctg acc ggg ctg ttc ggc gag gag gac 976
   Tyr Cys Leu Cys Arg Gly Cys Leu Thr Gly Leu Phe Gly Glu Glu Asp
   120 125 130 135
   gtg cgc ttc cgc agc gcc cct gtc tac atg ccc acc gtc gtc ctg cgc 1024
   Val Arg Phe Arg Ser Ala Pro Val Tyr Met Pro Thr Val Val Leu Arg
   140 145 150
   cgc acc ccc gcc tgc gcc ggc ggc cgt tcc gtc tac acc gag gcc tac 1072
   Arg Thr Pro Ala Cys Ala Gly Gly Arg Ser Val Tyr Thr Glu Ala Tyr
   155 160 165
   gtc acc atc ccc gtg ggc tgc acc tgc gtc ccc gag ccg gag aag gac 1120
   Val Thr Ile Pro Val Gly Cys Thr Cys Val Pro Glu Pro Glu Lys Asp
   170 175 180
   gca gac agc atc aac tcc agc atc gac aaa cag ggc gcc aag ctc ctg 1168
   Ala Asp Ser Ile Asn Ser Ser Ile Asp Lys Gln Gly Ala Lys Leu Leu
   185 190 195
   ctg ggc ccc aac gac gcg ccc gct ggc ccc tga g gccggtcctg 1212
   Leu Gly Pro Asn Asp Ala Pro Ala Gly Pro *
   200 205
   ccccgggagg tctccccggc ccgcatcccg aggcgcccaa gctggagccg cctggagggc 1272
   tcggtcggcg acctctgaag agagtgcacc gagcaaacca agtgccggag caccagcgcc 1332
   gcctttccat ggagactcgt aagcagcttc atctgacacg ggaatccctg gcttgctttt 1392
   agctacaagc aagcagcgtg gctggaagct gatgggaaac gacccggcac gggcatcctg 1452
   tgtgcggccc gcatggaggg tttggaaaag ttcacggagg ctccctgagg agcctctcag 1512
   atcggctgct gcgggtgcag ggcgtgactc accgctgggt gcttgccaaa gagataggga 1572
   cgcatatgct ttttaaagca atctaaaaat aataataagt atagcgacta tatacctact 1632
   tttaaaatca actgttttga atagaggcag agctatttta tattatcaaa tgagagctac 1692
   tctgttacat ttcttaacat ataaacatcg ttttttactt cttctggtag aattttttaa 1752
   agcataattg gaatccttgg ataaattttg tagctggtac actctggcct gggtctctga 1812
   attcagcctg tcaccgatgg ctgactgatg aaatggacac gtctcatctg acccactctt 1872
   ccttccactg aaggtcttca cgggcctcca ggtggaccaa agggatgcac aggcggctcg 1932
   catgccccag ggccagctaa gagttccaaa gatctcagat ttggttttag tcatgaatac 1992
   ataaacagtc tcaaactcgc acaatttttt cccccttttg aaagccactg gggccaattt 2052
   gtggttaaga ggtggtgaga taagaagtgg aacgtgacat ctttgccagt tgtcagaaga 2112
   atccaagcag gtattggctt agttgtaagg gctttaggat caggccgaat atgaggacaa 2172
   agtgggccac gttagcatct gcagagatca atctggaggc ttctgtttct gcattctgcc 2232
   acgagagcta ggtccttgat cttttcttta gattgaaagt ctgtctctga acacaattat 2292
   ttgtaaaagt tagaagttct tttttaaatc attaaaagag gcttgctgaa aaaaaaaaaa 2352
   aaaaaaaaa
   2361
<210> 7
   <211> 209
   <212> PRT
   <213> Homo sapiens
<400> 7
   Met Leu Gly Ala Leu Val Trp Met Leu Val Ala Gly Phe Leu Leu Ala
   1 5 10 15
   Leu Pro Pro Ser Trp Ala Ala Gly Ala Pro Arg Ala Gly Arg Arg Pro
   20 25 30
   Ala Arg Pro Arg Gly Cys Ala Asp Arg Pro Glu Glu Leu Leu Glu Gln
   35 40 45
   Leu Tyr Gly Arg Leu Ala Ala Gly Val Leu Ser Ala Phe His His Thr
   50 55 60
   Leu Gln Leu Gly Pro Arg Glu Gln Ala Arg Asn Ala Ser Cys Pro Ala
   65 70 75 80
   Gly Gly Arg Pro Ala Asp Arg Arg Phe Arg Pro Pro Thr Asn Leu Arg
                       85                      90                      95
   Ser Val Ser Pro Trp Ala Tyr Arg Ile Ser Tyr Asp Pro Ala Arg Tyr
                   100                     105                     110
   Pro Arg Tyr Leu Pro Glu Ala Tyr Cys Leu Cys Arg Gly Cys Leu Thr
               115                     120                     125
   Gly Leu Phe Gly Glu Glu Asp Val Arg Phe Arg Ser Ala Pro Val Tyr
       130                     135                     140
   Met Pro Thr Val Val Leu Arg Arg Thr Pro Ala Cys Ala Gly Gly Arg
   145                     150                     155                     160
   Ser Val Tyr Thr Glu Ala Tyr Val Thr Ile Pro Val Gly Cys Thr Cys
                       165                     170                     175
   Val Pro Glu Pro Glu Lys Asp Ala Asp Ser Ile Asn Ser Ser Ile Asp
                   180                     185                     190
   Lys Gln Gly Ala Lys Leu Leu Leu Gly Pro Asn Asp Ala Pro Ala Gly
               195                     200                     205
   Pro
<210> 8
   <211> 187
   <212> PRT
   <213> Homo sapiens
<400> 8
   Ala Gly Ala Pro Arg Ala Gly Arg Arg Pro Ala Arg Pro Arg Gly Cys
    1               5                  10                  15
   Ala Asp Arg Pro Glu Glu Leu Leu Glu Gln Leu Tyr Gly Arg Leu Ala
               20                  25                  30
   Ala Gly Val Leu Ser Ala Phe His His Thr Leu Gln Leu Gly Pro Arg
           35                  40                  45
   Glu Gln Ala Arg Asn Ala Ser Cys Pro Ala Gly Gly Arg Pro Ala Asp
       50                  55                  60
   Arg Arg Phe Arg Pro Pro Thr Asn Leu Arg Ser Val Ser Pro Trp Ala
   65                  70                  75                  80
   Tyr Arg Ile Ser Tyr Asp Pro Ala Arg Tyr Pro Arg Tyr Leu Pro Glu
                   85                  90                  95
   Ala Tyr Cys Leu Cys Arg Gly Cys Leu Thr Gly Leu Phe Gly Glu Glu
               100                 105                 110
   Asp Val Arg Phe Arg Ser Ala Pro Val Tyr Met Pro Thr Val Val Leu
           115                 120                 125
   Arg Arg Thr Pro Ala Cys Ala Gly Gly Arg Ser Val Tyr Thr Glu Ala
       130                 135                 140
   Tyr Val Thr Ile Pro Val Gly Cys Thr Cys Val Pro Glu Pro Glu Lys
   145                 150                 155                 160
   Asp Ala Asp Ser Ile Asn Ser Ser Ile Asp Lys Gln Gly Ala Lys Leu
                   165                 170                 175
   Leu Leu Gly Pro Asn Asp Ala Pro Ala Gly Pro
               180                 185
<210> 9
   <211> 1221
   <212> DNA
   <213> Mus musculis
<220>
   <221> CDS
   <222> (79)...(693)
<400> 9
   gggtgtcgcc cttatttact tcgcagaaga gccttcagcc cccctcctaa caagtctgga      60
   aagcatcacg gcgacgcg atg ttg ggg aca ctg gtc tgg atg ctc gcg gtc       111
                       Met Leu Gly Thr Leu Val Trp Met Leu Ala Val
                        1               5                  10
   ggc ttc ctg ctg gca ctg gcg ccg ggc cgc gcg gcg ggc gcg ctg agg       159
   Gly Phe Leu Leu Ala Leu Ala Pro Gly Arg Ala Ala Gly Ala Leu Arg
               15                  20                  25
   acc ggg agg cgc ccg gcg cgg ccg cgg gac tgc gcg gac cgg ccg gag       207
   Thr Gly Arg Arg Pro Ala Arg Pro Arg Asp Cys Ala Asp Arg Pro Glu
           30                  35                  40
   gag ctc ctg gag cag ctg tac ggg cgg ctg gcg gcc ggc gtg ctc agc       255
   Glu Leu Leu Glu Gln Leu Tyr Gly Arg Leu Ala Ala Gly Val Leu Ser
       45                  50                  55
   gcc ttc cac cac acg ctg cag ctc ggg ccg cgc gag cag gcg cgc aat       303
   Ala Phe His His Thr Leu Gln Leu Gly Pro Arg Glu Gln Ala Arg Asn
    60                  65                  70                  75
   gcc agc tgc ccg gcc ggg ggc agg gcc gcc gac cgc cgc ttc cgg cca       351
   Ala Ser Cys Pro Ala Gly Gly Arg Ala Ala Asp Arg Arg Phe Arg Pro
                   80                  85                  90
   ccc acc aac ctg cgc agc gtg tcg ccc tgg gcg tac agg att tcc tac       399
   Pro Thr Asn Leu Arg Ser Val Ser Pro Trp Ala Tyr Arg Ile Ser Tyr
                       95                  100                 105
   gac cct gct cgc ttt ccg agg tac ctg ccc gaa gcc tac tgc ctg tgc        447
   Asp Pro Ala Arg Phe Pro Arg Tyr Leu Pro Glu Ala Tyr Cys Leu Cys
               110                 115                 120
   cga ggc tgc ctg acc ggg ctc tac ggg gag gag gac ttc cgc ttt cgc        495
   Arg Gly Cys Leu Thr Gly Leu Tyr Gly Glu Glu Asp Phe Arg Phe Arg
           125                 130                 135
   agc aca ccc gtc ttc tct cca gcc gtg gtg ctg cgg cgc aca gcg gcc        543
   Ser Thr Pro Val Phe Ser Pro Ala Val Val Leu Arg Arg Thr Ala Ala
       140                 145                 150                 155
   tgc gcg ggc ggc cgc tct gtg tac gcc gaa cac tac atc acc atc ccg        591
   Cys Ala Gly Gly Arg Ser Val Tyr Ala Glu His Tyr Ile Thr Ile Pro
                   160                 165                 170
   gtg ggc tgc acc tgc gtg ccc gag ccg gac aag tcc gcg gac agt gcg        639
   Val Gly Cys Thr Cys Val Pro Glu Pro Asp Lys Ser Ala Asp Ser Ala
               175                 180                 185
   aac tcc agc atg gac aag ctg ctg ctg ggg ccc gcc gac agg cct gcg        687
   Asn Ser Ser Met Asp Lys Leu Leu Leu Gly Pro Ala Asp Arg Pro Ala
           190                 195                 200
   ggg cgc tgatgccggg gactgcccgc catggcccag cttcctgcat gcatcaggtc          743
   Gly Arg
           205
   ccctggccct gacaaaaccc accccatgat ccctggccgc tgcctaattt ttccaaaagg     803
   acagctacat aagctttaaa tatatttttc aaagtagaca ctacatatct acaactattt     863
   tgaatagtgg cagaaactat tttcatatta gtaatttaga gcaagcatgt tgtttttaaa     923
   cttctttgat atacaagcac atcacacaca tcccgttttc ctctagtagg attcttgagt     983
   gcataattgt agtgctcaga tgaacttcct tctgctgcac tgtgccctgt ccctgagtct    1043
   ctcctgtggc ccaagcttac taaggtgata atgagtgctc cggatctggg cacctaaggt    1103
   ctccaggtcc ctggagaggg agggatgtgg gggggctaga accaagcgcc cctttgttct    1163
   ttagcttatg gatggtctta actttataaa gattaaagtt tttggtgtta ttctttca      1221
<210> 10
   <211> 205
   <212> PRT
   <213> Mus musculis
<400> 10
   Met Leu Gly Thr Leu Val Trp Met Leu Ala Val Gly Phe Leu Leu Ala
    1               5                  10                  15
   Leu Ala Pro Gly Arg Ala Ala Gly Ala Leu Arg Thr Gly Arg Arg Pro
               20                  25                  30
   Ala Arg Pro Arg Asp Cys Ala Asp Arg Pro Glu Glu Leu Leu Glu Gln
           35                  40                  45
   Leu Tyr Gly Arg Leu Ala Ala Gly Val Leu Ser Ala Phe His His Thr
       50                  55                  60
   Leu Gln Leu Gly Pro Arg Glu Gln Ala Arg Asn Ala Ser Cys Pro Ala
   65                  70                  75                  80
   Gly Gly Arg Ala Ala Asp Arg Arg Phe Arg Pro Pro Thr Asn Leu Arg
                   85                  90                  95
   Ser Val Ser Pro Trp Ala Tyr Arg Ile Ser Tyr Asp Pro Ala Arg Phe
               100                 105                 110
   Pro Arg Tyr Leu Pro Glu Ala Tyr Cys Leu Cys Arg Gly Cys Leu Thr
           115                 120                 125
   Gly Leu Tyr Gly Glu Glu Asp Phe Arg Phe Arg Ser Thr Pro Val Phe
       130                 135                 140
   Ser Pro Ala Val Val Leu Arg Arg Thr Ala Ala Cys Ala Gly Gly Arg
   145                 150                 155                 160
   Ser Val Tyr Ala Glu His Tyr Ile Thr Ile Pro Val Gly Cys Thr Cys
                   165                 170                 175
   Val Pro Glu Pro Asp Lys Ser Ala Asp Ser Ala Asn Ser Ser Met Asp
               180                 185                 190
   Lys Leu Leu Leu Gly Pro Ala Asp Arg Pro Ala Gly Arg
           195                 200                 205
<210> 11
   <211> 183
   <212> PRT
   <213> Mus musculis
<400> 11
   Ala Gly Ala Leu Arg Thr Gly Arg Arg Pro Ala Arg Pro Arg Asp Cys
    1               5                  10                  15
   Ala Asp Arg Pro Glu Glu Leu Leu Glu Gln Leu Tyr Gly Arg Leu Ala
               20                  25                  30
   Ala Gly Val Leu Ser Ala Phe His His Thr Leu Gln Leu Gly Pro Arg
           35                  40                  45
   Glu Gln Ala Arg Asn Ala Ser Cys Pro Ala Gly Gly Arg Ala Ala Asp
       50                  55                  60
   Arg Arg Phe Arg Pro Pro Thr Asn Leu Arg Ser Val Ser Pro Trp Ala
   65                  70                  75                  80
   Tyr Arg Ile Ser Tyr Asp Pro Ala Arg Phe Pro Arg Tyr Leu Pro Glu
                   85                  90                  95
   Ala Tyr Cys Leu Cys Arg Gly Cys Leu Thr Gly Leu Tyr Gly Glu Glu
               100                 105                 110
   Asp Phe Arg Phe Arg Ser Thr Pro Val Phe Ser Pro Ala Val Val Leu
           115                 120                 125
   Arg Arg Thr Ala Ala Cys Ala Gly Gly Arg Ser Val Tyr Ala Glu His
       130                 135                 140
   Tyr Ile Thr Ile Pro Val Gly Cys Thr Cys Val Pro Glu Pro Asp Lys
   145                 150                 155                 160
   Ser Ala Asp Ser Ala Asn Ser Ser Met Asp Lys Leu Leu Leu Gly Pro
                   165                 170                 175
   Ala Asp Arg Pro Ala Gly Arg
               180
<210> 12
   <211> 3016
   <212> DNA
   <213> Homo sapiens
<220>
   <221> CDS
   <222> (12)...(2864)
<400> 12
   tggattacac a atg aca tgg aga atg gga ccc cgt ttc act atg ctg ttg      50
                Met Thr Trp Arg Met Gly Pro Arg Phe Thr Met Leu Leu
                 1               5                  10
   gcc atg tgg cta gtg tgt gga tca gaa ccc cac ccc cat gcc act att       98
   Ala Met Trp Leu Val Cys Gly Ser Glu Pro His Pro His Ala Thr Ile
        15                  20                  25
   aga ggc agc cac gga gga cgg aaa gtg cct ttg gtt tct ccg gac agc      146
   Arg Gly Ser His Gly Gly Arg Lys Val Pro Leu Val Ser Pro Asp Ser
    30                  35                  40                  45
   agt agg cca gct cgg ttt ctg agg cac act ggg agg tct cgc gga att      194
   Ser Arg Pro Ala Arg Phe Leu Arg His Thr Gly Arg Ser Arg Gly Ile
                   50                  55                  60
   gag aga tcc act ctg gag gaa cca aac ctt cag cct ctc cag aga agg      242
   Glu Arg Ser Thr Leu Glu Glu Pro Asn Leu Gln Pro Leu Gln Arg Arg
                   65                  70                  75
   agg agt gtg ccc gtg ttg aga cta gct cgc cca aca gag ccg cca gcc      290
   Arg Ser Val Pro Val Leu Arg Leu Ala Arg Pro Thr Glu Pro Pro Ala
               80                  85                  90
   cgc tcg gac atc aat ggg gcc gcc gtg aga cct gag caa aga cca gca      338
   Arg Ser Asp Ile Asn Gly Ala Ala Val Arg Pro Glu Gln Arg Pro Ala
           95                  100                 105
   gcc agg ggc tct ccg cgt gag atg atc aga gat gag ggg tcc tca gct      386
   Ala Arg Gly Ser Pro Arg Glu Met Ile Arg Asp Glu Gly Ser Ser Ala
   110                 115                 120                 125
   cgg tca aga atg ttg cgt ttc cct tcg ggg tcc agc tct ccc aac atc      434
   Arg Ser Arg Met Leu Arg Phe Pro Ser Gly Ser Ser Ser Pro Asn Ile
                       130                 135                 140
   ctt gcc agc ttt gca ggg aag aac aga gta tgg gtc atc tca gcc cct      482
   Leu Ala Ser Phe Ala Gly Lys Asn Arg Val Trp Val Ile Ser Ala Pro
                   145                 150                 155
   cat gcc tcg gaa ggc tac tac cgc ctc atg atg agc ctg ctg aag gac      530
   His Ala Ser Glu Gly Tyr Tyr Arg Leu Met Met Ser Leu Leu Lys Asp
               160                 165                 170
   gat gtg tac tgt gag ctg gcg gag agg cac atc caa cag att gtg ctc      578
   Asp Val Tyr Cys Glu Leu Ala Glu Arg His Ile Gln Gln Ile Val Leu
           175                 180                 185
   ttc cac cag gca ggt gag gaa gga ggc aag gtg aga agg atc acc agc      626
   Phe His Gln Ala Gly Glu Glu Gly Gly Lys Val Arg Arg Ile Thr Ser
   190                 195                 200                 205
   gag ggc cag atc ctg gag cag ccc ctg gac cct agc ctc atc cct aag      674
   Glu Gly Gln Ile Leu Glu Gln Pro Leu Asp Pro Ser Leu Ile Pro Lys
                       210                 215                 220
   ctg atg agc ttc ctg aag ctg gag aag ggc aag ttt ggc atg gtg ctg      722
   Leu Met Ser Phe Leu Lys Leu Glu Lys Gly Lys Phe Gly Met Val Leu
                   225                 230                 235
   ctg aag aag acg ctg cag gtg gag gag cgc tat cca tat ccc gtt agg      770
   Leu Lys Lys Thr Leu Gln Val Glu Glu Arg Tyr Pro Tyr Pro Val Arg
               240                 245                 250
   ctg gaa gcc atg tac gag gtc atc gac caa ggc ccc atc cgt agg atc      818
   Leu Glu Ala Met Tyr Glu Val Ile Asp Gln Gly Pro Ile Arg Arg Ile
           255                 260                 265
   gag aag atc agg cag aag ggc ttt gtc cag aaa tgt aag gcc tct ggt      866
   Glu Lys Ile Arg Gln Lys Gly Phe Val Gln Lys Cys Lys Ala Ser Gly
   270                 275                 280                 285
   gta gag ggc cag gtg gtg gcg gag ggg aat gac ggt gga ggg gga gca      914
   Val Glu Gly Gln Val Val Ala Glu Gly Asn Asp Gly Gly Gly Gly Ala
                   290                 295                 300
   gga agg cca agc ctg ggc agc gag aag aag aaa gag gac cca agg aga      962
   Gly Arg Pro Ser Leu Gly Ser Glu Lys Lys Lys Glu Asp Pro Arg Arg
               305                 310                 315
   gca caa gtc cca cca acc aga gag agt cgg gtg aag gtc ctg aga aaa     1010
   Ala Gln Val Pro Pro Thr Arg Glu Ser Arg Val Lys Val Leu Arg Lys
           320                 325                 330
   ctg gcc gcc act gca cca gct ttg ccc caa cct ccc tca acc ccc aga     1058
   Leu Ala Ala Thr Ala Pro Ala Leu Pro Gln Pro Pro Ser Thr Pro Arg
       335                 340                 345
   gcc acc acc ctt cct cct gcc cca gcc aca aca gtg act cgg tcc acg     1106
   Ala Thr Thr Leu Pro Pro Ala Pro Ala Thr Thr Val Thr Arg Ser Thr
   350                 355                 360                 365
   tcc cgg gcg gta aca gtt gct gca aga cct atg acc acc act gcc ttt     1154
   Ser Arg Ala Val Thr Val Ala Ala Arg Pro Met Thr Thr Thr Ala Phe
                   370                 375                 380
   ccc acc acg cag agg ccc tgg acc ccc tca ccc tcc cac agg ccc cct     1202
   Pro Thr Thr Gln Arg Pro Trp Thr Pro Ser Pro Ser His Arg Pro Pro
               385                 390                 395
   aca acc act gag gtg atc act gcc agg aga ccc tca gtt tca gag aat     1250
   Thr Thr Thr Glu Val Ile Thr Ala Arg Arg Pro Ser Val Ser Glu Asn
           400                     405                     410
   ctt tac cct cca tcc cgg aag gat cag cac agg gag agg cca cag aca      1298
   Leu Tyr Pro Pro Ser Arg Lys Asp Gln His Arg Glu Arg Pro Gln Thr
       415                     420                     425
   acc agg agg ccc agc aag gcc acc agc ttg gag agc ttc aca aat gcc      1346
   Thr Arg Arg Pro Ser Lys Ala Thr Ser Leu Glu Ser Phe Thr Asn Ala
   430                     435                     440                     445
   cct ccc acc acc atc tca gaa ccc agc aca agg gct gct ggc cca ggc      1394
   Pro Pro Thr Thr Ile Ser Glu Pro Ser Thr Arg Ala Ala Gly Pro Gly
                   450                     455                     460
   cgt ttc cgg gac aac cgc atg gac agg cgg gaa cat ggc cac cga gac      1442
   Arg Phe Arg Asp Asn Arg Met Asp Arg Arg Glu His Gly His Arg Asp
               465                     470                     475
   cca aat gtg gtg cca ggt cct ccc aag cca gca aag gag aaa cct ccc      1490
   Pro Asn Val Val Pro Gly Pro Pro Lys Pro Ala Lys Glu Lys Pro Pro
           480                     485                     490
   aaa aag aag gcc cag gac aaa att ctt agt aat gag tat gag gag aag      1538
   Lys Lys Lys Ala Gln Asp Lys Ile Leu Ser Asn Glu Tyr Glu Glu Lys
       495                     500                     505
   tat gac ctc agc cgg cct act gcc tct cag ctg gag gac gag ctg cag      1586
   Tyr Asp Leu Ser Arg Pro Thr Ala Ser Gln Leu Glu Asp Glu Leu Gln
   510                     515                     520                     525
   gtg ggg aat gtt ccc ctt aaa aaa gca aag gag tct aaa aag cat gaa      1634
   Val Gly Asn Val Pro Leu Lys Lys Ala Lys Glu Ser Lys Lys His Glu
                   530                     535                     540
   aag ctt gag aaa cca gag aag gag aag aaa aaa aag atg aag aat gag      1682
   Lys Leu Glu Lys Pro Glu Lys Glu Lys Lys Lys Lys Met Lys Asn Glu
               545                     550                     555
   aac gca gac aag tta ctt aag agt gaa aag caa atg aag aag tct gag      1730
   Asn Ala Asp Lys Leu Leu Lys Ser Glu Lys Gln Met Lys Lys Ser Glu
           560                     565                     570
   aaa aag agc aag caa gag aaa gag aag agc aag aag aaa aaa gga ggt      1778
   Lys Lys Ser Lys Gln Glu Lys Glu Lys Ser Lys Lys Lys Lys Gly Gly
   575 580 585
   aaa aca gaa cag gat ggc tat cag aaa ccc acc aac aaa cac ttc acg 1826
   Lys Thr Glu Gln Asp Gly Tyr Gln Lys Pro Thr Asn Lys His Phe Thr
   590 595 600 605
   cag agt ccc aag aag tca gtg gcc gac ctg ctg ggg tcc ttt gaa ggc 1874
   Gln Ser Pro Lys Lys Ser Val Ala Asp Leu Leu Gly Ser Phe Glu Gly
   610 615 620
   aaa cga aga ctc ctt ctg atc act gct ccc aag gct gag aac aat atg 1922
   Lys Arg Arg Leu Leu Leu Ile Thr Ala Pro Lys Ala Glu Asn Asn Met
   625 630 635
   tat gtg caa caa cgt gat gaa tat ctg gaa agt ttc tgc aag atg gct 1970
   Tyr Val Gln Gln Arg Asp Glu Tyr Leu Glu Ser Phe Cys Lys Met Ala
   640 645 650
   acc agg aaa atc tct gtg atc acc atc ttc ggc cct gtc aac aac agc 2018
   Thr Arg Lys Ile Ser Val Ile Thr Ile Phe Gly Pro Val Asn Asn Ser
   655 660 665
   acc atg aaa atc gac cac ttt cag cta gat aat gag aag ccc atg cga 2066
   Thr Met Lys Ile Asp His Phe Gln Leu Asp Asn Glu Lys Pro Met Arg
   670 675 680 685
   gtg gtg gat gat gaa gac ttg gta gac cag cgt ctc atc agc gag ctg 2114
   Val Val Asp Asp Glu Asp Leu Val Asp Gln Arg Leu Ile Ser Glu Leu
   690 695 700
   agg aaa gag tac gga atg acc tac aat gac ttc ttc atg gtg cta aca 2162
   Arg Lys Glu Tyr Gly Met Thr Tyr Asn Asp Phe Phe Met Val Leu Thr
   705 710 715
   gat gtg gat ctg aga gtc aag caa tac tat gag gta cca ata aca atg 2210
   Asp Val Asp Leu Arg Val Lys Gln Tyr Tyr Glu Val Pro Ile Thr Met
   720 725 730
   aag tct gtg ttt gat ctg atc gat act ttc cag tcc cga atc aaa gat 2258
   Lys Ser Val Phe Asp Leu Ile Asp Thr Phe Gln Ser Arg Ile Lys Asp
   735 740 745
   atg gag aag cag aag aag gag ggc att gtt tgc aaa gag gac aaa aag      2306
   Met Glu Lys Gln Lys Lys Glu Gly Ile Val Cys Lys Glu Asp Lys Lys
   750                 755                 760                 765
   cag tcc ctg gag aac ttc cta tcc agg ttc cgg tgg agg agg agg ttg      2354
   Gln Ser Leu Glu Asn Phe Leu Ser Arg Phe Arg Trp Arg Arg Arg Leu
                   770                 775                 780
   ctg gtg atc tct gct cct aac gat gaa gac tgg gcc tat tca cag cag      2402
   Leu Val Ile Ser Ala Pro Asn Asp Glu Asp Trp Ala Tyr Ser Gln Gln
               785                 790                 795
   ctc tct gcc ctc agt ggt cag gcg tgc aat ttt ggt ctg cgc cac ata      2450
   Leu Ser Ala Leu Ser Gly Gln Ala Cys Asn Phe Gly Leu Arg His Ile
           800                 805                 810
   acc att ctg aag ctt tta ggc gtt gga gag gaa gtt ggg gga gtg tta      2498
   Thr Ile Leu Lys Leu Leu Gly Val Gly Glu Glu Val Gly Gly Val Leu
       815                 820                 825
   gaa ctg ttc cca att aat ggg agc tct gtt gtt gag cga gaa gac gta      2546
   Glu Leu Phe Pro Ile Asn Gly Ser Ser Val Val Glu Arg Glu Asp Val
   830                 835                 840                 845
   cca gcc cat ttg gtg aaa gac att cgt aac tat ttt caa gtg agc ccg      2594
   Pro Ala His Leu Val Lys Asp Ile Arg Asn Tyr Phe Gln Val Ser Pro
                   850                 855                 860
   gag tac ttc tcc atg ctt cta gtc gga aaa gac gga aat gtc aaa tcc      2642
   Glu Tyr Phe Ser Met Leu Leu Val Gly Lys Asp Gly Asn Val Lys Ser
               865                 870                 875
   tgg tat cct tcc cca atg tgg tcc atg gtg att gtg tac gat tta att      2690
   Trp Tyr Pro Ser Pro Met Trp Ser Met Val Ile Val Tyr Asp Leu Ile
           880                 885                 890
   gat tcg atg caa ctt cgg aga cag gaa atg gcg att cag cag tca ctg      2738
   Asp Ser Met Gln Leu Arg Arg Gln Glu Met Ala Ile Gln Gln Ser Leu
       895                 900                 905
   ggg atg cgc tgc cca gaa gat gag tat gca ggc tat ggt tac cat agt      2786
   Gly Met Arg Cys Pro Glu Asp Glu Tyr Ala Gly Tyr Gly Tyr His Ser
   910                 915                 920                 925
   tac cac caa gga tac cag gat ggt tac cag gat gac tac cgt cat cat      2834
   Tyr His Gln Gly Tyr Gln Asp Gly Tyr Gln Asp Asp Tyr Arg His His
                       930                 935                 940
   gag agt tat cac cat gga tac cct tac tga gcagaaatat gtaaccttag       2884
   Glu Ser Tyr His His Gly Tyr Pro Tyr  *
                   945                 950
   actcagccag tttcctctgc agctgctaaa actacatgtg gccagctcca ttcttccaca   2944
   ctgcgtacta catttcctgc ctttttcttt cagtgttttt ctaagactaa ataaatagca   3004
   aactttcacc ta
   3016
<210> 13
   <211> 950
   <212> PRT
   <213> Homo sapiens
<400> 13
   Met Thr Trp Arg Met Gly Pro Arg Phe Thr Met Leu Leu Ala Met Trp
    1               5                  10                  15
   Leu Val Cys Gly Ser Glu Pro His Pro His Ala Thr Ile Arg Gly Ser
               20                  25                  30
   His Gly Gly Arg Lys Val Pro Leu Val Ser Pro Asp Ser Ser Arg Pro
           35                  40                  45
   Ala Arg Phe Leu Arg His Thr Gly Arg Ser Arg Gly Ile Glu Arg Ser
       50                  55                  60
   Thr Leu Glu Glu Pro Asn Leu Gln Pro Leu Gln Arg Arg Arg Ser Val
   65                  70                  75                  80
   Pro Val Leu Arg Leu Ala Arg Pro Thr Glu Pro Pro Ala Arg Ser Asp
                   85                  90                  95
   Ile Asn Gly Ala Ala Val Arg Pro Glu Gln Arg Pro Ala Ala Arg Gly
               100                 105                 110
   Ser Pro Arg Glu Met Ile Arg Asp Glu Gly Ser Ser Ala Arg Ser Arg
           115                 120                 125
   Met Leu Arg Phe Pro Ser Gly Ser Ser Ser Pro Asn Ile Leu Ala Ser
       130                 135                 140
   Phe Ala Gly Lys Asn Arg Val Trp Val Ile Ser Ala Pro His Ala Ser
   145                 150                 155                 160
   Glu Gly Tyr Tyr Arg Leu Met Met Ser Leu Leu Lys Asp Asp Val Tyr
                   165                 170                 175
   Cys Glu Leu Ala Glu Arg His Ile Gln Gln Ile Val Leu Phe His Gln
   180 185 190
   Ala Gly Glu Glu Gly Gly Lys Val Arg Arg Ile Thr Ser Glu Gly Gln
   195 200 205
   Ile Leu Glu Gln Pro Leu Asp Pro Ser Leu Ile Pro Lys Leu Met Ser
   210 215 220
   Phe Leu Lys Leu Glu Lys Gly Lys Phe Gly Met Val Leu Leu Lys Lys
   225 230 235 240
   Thr Leu Gln Val Glu Glu Arg Tyr Pro Tyr Pro Val Arg Leu Glu Ala
   245 250 255
   Met Tyr Glu Val Ile Asp Gln Gly Pro Ile Arg Arg Ile Glu Lys Ile
   260 265 270
   Arg Gln Lys Gly Phe Val Gln Lys Cys Lys Ala Ser Gly Val Glu Gly
   275 280 285
   Gln Val Val Ala Glu Gly Asn Asp Gly Gly Gly Gly Ala Gly Arg Pro
   290 295 300
   Ser Leu Gly Ser Glu Lys Lys Lys Glu Asp Pro Arg Arg Ala Gln Val
   305 310 315 320
   Pro Pro Thr Arg Glu Ser Arg Val Lys Val Leu Arg Lys Leu Ala Ala
   325 330 335
   Thr Ala Pro Ala Leu Pro Gln Pro Pro Ser Thr Pro Arg Ala Thr Thr
   340 345 350
   Leu Pro Pro Ala Pro Ala Thr Thr Val Thr Arg Ser Thr Ser Arg Ala
   355 360 365
   Val Thr Val Ala Ala Arg Pro Met Thr Thr Thr Ala Phe Pro Thr Thr
   370 375 380
   Gln Arg Pro Trp Thr Pro Ser Pro Ser His Arg Pro Pro Thr Thr Thr
   385 390 395 400
   Glu Val Ile Thr Ala Arg Arg Pro Ser Val Ser Glu Asn Leu Tyr Pro
   405 410 415
   Pro Ser Arg Lys Asp Gln His Arg Glu Arg Pro Gln Thr Thr Arg Arg
   420 425 430
   Pro Ser Lys Ala Thr Ser Leu Glu Ser Phe Thr Asn Ala Pro Pro Thr
   435 440 445
   Thr Ile Ser Glu Pro Ser Thr Arg Ala Ala Gly Pro Gly Arg Phe Arg
   450 455 460
   Asp Asn Arg Met Asp Arg Arg Glu His Gly His Arg Asp Pro Asn Val
   465 470 475 480
   Val Pro Gly Pro Pro Lys Pro Ala Lys Glu Lys Pro Pro Lys Lys Lys
   485 490 495
   Ala Gln Asp Lys Ile Leu Ser Asn Glu Tyr Glu Glu Lys Tyr Asp Leu
   500 505 510
   Ser Arg Pro Thr Ala Ser Gln Leu Glu Asp Glu Leu Gln Val Gly Asn
   515 520 525
   Val Pro Leu Lys Lys Ala Lys Glu Ser Lys Lys His Glu Lys Leu Glu
   530 535 540
   Lys Pro Glu Lys Glu Lys Lys Lys Lys Met Lys Asn Glu Asn Ala Asp
   545 550 555 560
   Lys Leu Leu Lys Ser Glu Lys Gln Met Lys Lys Ser Glu Lys Lys Ser
   565 570 575
   Lys Gln Glu Lys Glu Lys Ser Lys Lys Lys Lys Gly Gly Lys Thr Glu
   580 585 590
   Gln Asp Gly Tyr Gln Lys Pro Thr Asn Lys His Phe Thr Gln Ser Pro
   595 600 605
   Lys Lys Ser Val Ala Asp Leu Leu Gly Ser Phe Glu Gly Lys Arg Arg
   610 615 620
   Leu Leu Leu Ile Thr Ala Pro Lys Ala Glu Asn Asn Met Tyr Val Gln
   625 630 635 640
   Gln Arg Asp Glu Tyr Leu Glu Ser Phe Cys Lys Met Ala Thr Arg Lys
   645 650 655
   Ile Ser Val Ile Thr Ile Phe Gly Pro Val Asn Asn Ser Thr Met Lys
   660 665 670
   Ile Asp His Phe Gln Leu Asp Asn Glu Lys Pro Met Arg Val Val Asp
   675 680 685
   Asp Glu Asp Leu Val Asp Gln Arg Leu Ile Ser Glu Leu Arg Lys Glu
   690 695 700
   Tyr Gly Met Thr Tyr Asn Asp Phe Phe Met Val Leu Thr Asp Val Asp
   705 710 715 720
   Leu Arg Val Lys Gln Tyr Tyr Glu Val Pro Ile Thr Met Lys Ser Val
   725 730 735
   Phe Asp Leu Ile Asp Thr Phe Gln Ser Arg Ile Lys Asp Met Glu Lys
   740 745 750
   Gln Lys Lys Glu Gly Ile Val Cys Lys Glu Asp Lys Lys Gln Ser Leu
   755 760 765
   Glu Asn Phe Leu Ser Arg Phe Arg Trp Arg Arg Arg Leu Leu Val Ile
   770 775 780
   Ser Ala Pro Asn Asp Glu Asp Trp Ala Tyr Ser Gln Gln Leu Ser Ala
   785 790 795 800
   Leu Ser Gly Gln Ala Cys Asn Phe Gly Leu Arg His Ile Thr Ile Leu
   805 810 815
   Lys Leu Leu Gly Val Gly Glu Glu Val Gly Gly Val Leu Glu Leu Phe
   820 825 830
   Pro Ile Asn Gly Ser Ser Val Val Glu Arg Glu Asp Val Pro Ala His
   835 840 845
   Leu Val Lys Asp Ile Arg Asn Tyr Phe Gln Val Ser Pro Glu Tyr Phe
   850 855 860
   Ser Met Leu Leu Val Gly Lys Asp Gly Asn Val Lys Ser Trp Tyr Pro
   865 870 875 880
   Ser Pro Met Trp Ser Met Val Ile Val Tyr Asp Leu Ile Asp Ser Met
   885 890 895
   Gln Leu Arg Arg Gln Glu Met Ala Ile Gln Gln Ser Leu Gly Met Arg
   900 905 910
   Cys Pro Glu Asp Glu Tyr Ala Gly Tyr Gly Tyr His Ser Tyr His Gln
   915 920 925
   Gly Tyr Gln Asp Gly Tyr Gln Asp Asp Tyr Arg His His Glu Ser Tyr
   930 935 940
   His His Gly Tyr Pro Tyr
   945 950
<210> 14
   <211> 929
   <212> PRT
   <213> Homo sapiens
<400> 14
   Glu Pro His Pro His Ala Thr Ile Arg Gly Ser His Gly Gly Arg Lys
   1 5 10 15
   Val Pro Leu Val Ser Pro Asp Ser Ser Arg Pro Ala Arg Phe Leu Arg
   20 25 30
   His Thr Gly Arg Ser Arg Gly Ile Glu Arg Ser Thr Leu Glu Glu Pro
   35 40 45
   Asn Leu Gln Pro Leu Gln Arg Arg Arg Ser Val Pro Val Leu Arg Leu
   50 55 60
   Ala Arg Pro Thr Glu Pro Pro Ala Arg Ser Asp Ile Asn Gly Ala Ala
   65 70 75 80
   Val Arg Pro Glu Gln Arg Pro Ala Ala Arg Gly Ser Pro Arg Glu Met
   85 90 95
   Ile Arg Asp Glu Gly Ser Ser Ala Arg Ser Arg Met Leu Arg Phe Pro
   100 105 110
   Ser Gly Ser Ser Ser Pro Asn Ile Leu Ala Ser Phe Ala Gly Lys Asn
   115 120 125
   Arg Val Trp Val Ile Ser Ala Pro His Ala Ser Glu Gly Tyr Tyr Arg
   130 135 140
   Leu Met Met Ser Leu Leu Lys Asp Asp Val Tyr Cys Glu Leu Ala Glu
   145 150 155 160
   Arg His Ile Gln Gln Ile Val Leu Phe His Gln Ala Gly Glu Glu Gly
   165 170 175
   Gly Lys Val Arg Arg Ile Thr Ser Glu Gly Gln Ile Leu Glu Gln Pro
   180 185 190
   Leu Asp Pro Ser Leu Ile Pro Lys Leu Met Ser Phe Leu Lys Leu Glu
   195 200 205
   Lys Gly Lys Phe Gly Met Val Leu Leu Lys Lys Thr Leu Gln Val Glu
   210 215 220
   Glu Arg Tyr Pro Tyr Pro Val Arg Leu Glu Ala Met Tyr Glu Val Ile
   225 230 235 240
   Asp Gln Gly Pro Ile Arg Arg Ile Glu Lys Ile Arg Gln Lys Gly Phe
   245 250 255
   Val Gln Lys Cys Lys Ala Ser Gly Val Glu Gly Gln Val Val Ala Glu
   260 265 270
   Gly Asn Asp Gly Gly Gly Gly Ala Gly Arg Pro Ser Leu Gly Ser Glu
   275 280 285
   Lys Lys Lys Glu Asp Pro Arg Arg Ala Gln Val Pro Pro Thr Arg Glu
   290 295 300
   Ser Arg Val Lys Val Leu Arg Lys Leu Ala Ala Thr Ala Pro Ala Leu
   305 310 315 320
   Pro Gln Pro Pro Ser Thr Pro Arg Ala Thr Thr Leu Pro Pro Ala Pro
   325 330 335
   Ala Thr Thr Val Thr Arg Ser Thr Ser Arg Ala Val Thr Val Ala Ala
   340 345 350
   Arg Pro Met Thr Thr Thr Ala Phe Pro Thr Thr Gln Arg Pro Trp Thr
   355 360 365
   Pro Ser Pro Ser His Arg Pro Pro Thr Thr Thr Glu Val Ile Thr Ala
   370 375 380
   Arg Arg Pro Ser Val Ser Glu Asn Leu Tyr Pro Pro Ser Arg Lys Asp
   385 390 395 400
   Gln His Arg Glu Arg Pro Gln Thr Thr Arg Arg Pro Ser Lys Ala Thr
   405 410 415
   Ser Leu Glu Ser Phe Thr Asn Ala Pro Pro Thr Thr Ile Ser Glu Pro
   420 425 430
   Ser Thr Arg Ala Ala Gly Pro Gly Arg Phe Arg Asp Asn Arg Met Asp
   435 440 445
   Arg Arg Glu His Gly His Arg Asp Pro Asn Val Val Pro Gly Pro Pro
   450 455 460
   Lys Pro Ala Lys Glu Lys Pro Pro Lys Lys Lys Ala Gln Asp Lys Ile
   465 470 475 480
   Leu Ser Asn Glu Tyr Glu Glu Lys Tyr Asp Leu Ser Arg Pro Thr Ala
   485 490 495
   Ser Gln Leu Glu Asp Glu Leu Gln Val Gly Asn Val Pro Leu Lys Lys
   500 505 510
   Ala Lys Glu Ser Lys Lys His Glu Lys Leu Glu Lys Pro Glu Lys Glu
   515 520 525
   Lys Lys Lys Lys Met Lys Asn Glu Asn Ala Asp Lys Leu Leu Lys Ser
   530 535 540
   Glu Lys Gln Met Lys Lys Ser Glu Lys Lys Ser Lys Gln Glu Lys Glu
   545                 550                 555                 560
   Lys Ser Lys Lys Lys Lys Gly Gly Lys Thr Glu Gln Asp Gly Tyr Gln
                   565                 570                 575
   Lys Pro Thr Asn Lys His Phe Thr Gln Ser Pro Lys Lys Ser Val Ala
               580                 585                 590
   Asp Leu Leu Gly Ser Phe Glu Gly Lys Arg Arg Leu Leu Leu Ile Thr
           595                 600                 605
   Ala Pro Lys Ala Glu Asn Asn Met Tyr Val Gln Gln Arg Asp Glu Tyr
       610                 615                 620
   Leu Glu Ser Phe Cys Lys Met Ala Thr Arg Lys Ile Ser Val Ile Thr
   625                 630                 635                 640
   Ile Phe Gly Pro Val Asn Asn Ser Thr Met Lys Ile Asp His Phe Gln
                   645                 650                 655
   Leu Asp Asn Glu Lys Pro Met Arg Val Val Asp Asp Glu Asp Leu Val
               660                 665                 670
   Asp Gln Arg Leu Ile Ser Glu Leu Arg Lys Glu Tyr Gly Met Thr Tyr
           675                 680                 685
   Asn Asp Phe Phe Met Val Leu Thr Asp Val Asp Leu Arg Val Lys Gln
       690                 695                 700
   Tyr Tyr Glu Val Pro Ile Thr Met Lys Ser Val Phe Asp Leu Ile Asp
   705                 710                 715                 720
   Thr Phe Gln Ser Arg Ile Lys Asp Met Glu Lys Gln Lys Lys Glu Gly
                   725                 730                 735
   Ile Val Cys Lys Glu Asp Lys Lys Gln Ser Leu Glu Asn Phe Leu Ser
               740                 745                 750
   Arg Phe Arg Trp Arg Arg Arg Leu Leu Val Ile Ser Ala Pro Asn Asp
           755                 760                 765
   Glu Asp Trp Ala Tyr Ser Gln Gln Leu Ser Ala Leu Ser Gly Gln Ala
       770                 775                 780
   Cys Asn Phe Gly Leu Arg His Ile Thr Ile Leu Lys Leu Leu Gly Val
   785                 790                 795                 800
   Gly Glu Glu Val Gly Gly Val Leu Glu Leu Phe Pro Ile Asn Gly Ser
                   805                 810                 815
   Ser Val Val Glu Arg Glu Asp Val Pro Ala His Leu Val Lys Asp Ile
               820                 825                 830
   Arg Asn Tyr Phe Gln Val Ser Pro Glu Tyr Phe Ser Met Leu Leu Val
           835                 840                 845
   Gly Lys Asp Gly Asn Val Lys Ser Trp Tyr Pro Ser Pro Met Trp Ser
       850                 855                 860
   Met Val Ile Val Tyr Asp Leu Ile Asp Ser Met Gln Leu Arg Arg Gln
   865                 870                 875                 880
   Glu Met Ala Ile Gln Gln Ser Leu Gly Met Arg Cys Pro Glu Asp Glu
                           885                     890                     895
   Tyr Ala Gly Tyr Gly Tyr His Ser Tyr His Gln Gly Tyr Gln Asp Gly
                       900                     905                     910
   Tyr Gln Asp Asp Tyr Arg His His Glu Ser Tyr His His Gly Tyr Pro
                   915                     920                     925
   Tyr
<210> 15
   <211> 19
   <212> PRT
   <213> Homo sapiens
<400> 15
   Tyr Pro Ser Pro Met Trp Ser Met Val Ile Val Tyr Asp Leu Ile Asp
   1               5                   10                      15
   Ser Met Gln
<210> 16
   <211> 20
   <212> PRT
   <213> Homo sapiens
<400> 16
   Tyr Phe Gln Val Ser Pro Glu Tyr Phe Ser Met Leu Leu Val Gly Lys
   1               5                   10                      15
   Asp Gly Asn Val
               20